(19) Europäisches Patentamt
European Patent Office
Office européen des brevets

(11) Veröffentlichungsnummer: **0 164 573**
**B1**

(12) # EUROPÄISCHE PATENTSCHRIFT

(45) Veröffentlichungstag der Patentschrift: **09.08.89**

(21) Anmeldenummer: **85105620.0**

(22) Anmeldetag: **08.05.85**

(51) Int. Cl.⁴: **C 12 P 7/40**, C 12 P 41/00, C 12 N 1/14 // (C12N1/14, C12R1:66, 1:79),(C12P7/40, C12R1:38, 1:66, 1:79)

(54) **Verfahren zur Herstellung von Cyclopropancarbonsäuren.**

(30) Priorität: **17.05.84 DE 3418374**

(43) Veröffentlichungstag der Anmeldung: **18.12.85 Patentblatt 85/51**

(45) Bekanntmachung des Hinweises auf die Patenterteilung: **09.08.89 Patentblatt 89/32**

(84) Benannte Vertragsstaaten: **BE CH DE FR GB IT LI NL**

(56) Entgegenhaltungen:
**PESTICIDE BIOCHEMISTRY AND PHYSIOLOGY, Band 7, 1977, Seiten 391-401, Academic Press Inc.; D.M. SODERLUND et al.: "Effects of pyrethroid structure on rates of hydrolysis and oxidation by mouse liver microsomal enzymes"**
**CHEMICAL ABSTRACTS, Band 104, Nr. 21, Mai 1986, Seite 514, Nr. 184884m, Columbus, Ohio, US; & JP-A-60 199 393 (SUMITOMO CHEMICAL CO., LTD.) 08.10.1985**

(73) Patentinhaber: **BAYER AG**
**D-5090 Leverkusen 1 Bayerwerk (DE)**

(72) Erfinder: **Hildebrand, Heinz, Dr.**
**Nüller Strasse 83**
**D-5600 Wuppertal 1 (DE)**
Erfinder: **Zitzmann, Werner, Dr. Prof.**
**Burscheider Strasse 225**
**D-5090 Leverkusen 3 (DE)**
Erfinder: **Arlt, Dieter, Dr.**
**Rybniker Strasse 2**
**D-5000 Köln 80 (DE)**
Erfinder: **Kölbl, Heinz, Dr.**
**Andreas-Gryphius-Strasse 20**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 164 573 B1**

Jouve, 18, rue St-Denis, 75001 Paris, France

## Beschreibung

Die vorliegende Erfindung betrifft ein Verfahren zur Herstellung von (+)-trans-Cyclopropancarbonsäuren durch die Behandlung von (±)-cis/(±)-trans-Cyclopropancarbonsäureestern mit Hydrolasen mikrobieller Herkunft. Die Cyclopropancarbonsäuren können in hochwirksame insektizide Wirkstoffe (Pyrethroide) umgewandelt werden (vgl. z. B. K. Naumann, Chemie der Pflanzenschutz- und Schädlingsbekämpfungsmittel, herausgegeben von R. Wegler, Band 7, Springer-Verlag Berlin, Heidelberg, New York, 1981), wobei die (+)-trans-Verbindungen von besonderem praktischem Interesse sind, da sie zu besonders hochwirksamen insektiziden Pyrethroiden umgesetzt werden können.

Aus Pesticide Biochemistry and Physiology 7, 391-401 (1977) ist bereits bekannt geworden, daß Enzyme aus Mäuseleber bestimmte trans-Cyclopropancarbonsäureester rascher hydrolysieren können, als die entsprechenden cis-Verbindungen. Selbst wenn es möglich sein sollte, entsprechende Reaktionen für die Herstellung der besonders wertvollen (+)-trans-Cyclopropancarbonsäuren aufzufinden und heranzuziehen, erscheint die Verwendung von Säugetierenzymen für technische Zwecke praktisch nicht geeignet zu sein.

Es wurde nun gefunden, daß man (+)-trans-Cyclopropancarbonsäuren oder ihre Salze der allgemeinen Formel (I)

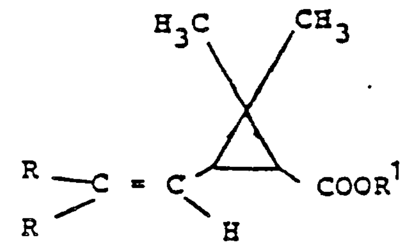

(I)

in welcher

R für Halogen oder $C_1$-$C_4$-Alkyl steht und

$R^1$ für Wasserstoff oder das Äquivalent eines Metallions steht,

und wobei in Formel (I) die sterischen Verhältnisse nicht wiedergegeben werden, erhält, wenn man die (±)-trans-Cyclopropancarbonsäureester der allgemeinen Formel (II), gegebenenfalls in Mischung mit den entsprechenden (±)-cis-Cyclopropancarbonsäureestern der allgemeinen Formel (II)

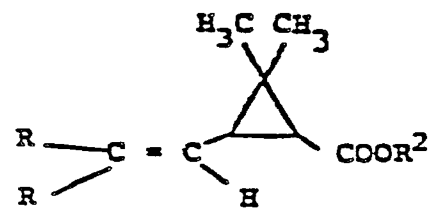

(II)

((±)-trans oder Gemisch aus (±)-trans und (±)-cis)

in welcher

R die bei der allgemeinen Formel (I) angegebene Bedeutung hat und

$R^2$ für Methyl oder Ethyl steht, wobei in Formel (II) die sterischen Verhältnisse nicht wiedergegeben werden,

mit Hydrolasen mikrobieller Herkunft, welche extrazellulär und/oder zellulär, gegebenenfalls immobilisiert, vorliegen, behandelt und die entstandenen (+)-trans-Cyclopropancarbonsäuren oder ihre Salze der allgemeinen Formel (I) nach den üblichen Methoden isoliert und gegebenenfalls reinigt und gegebenenfalls die Salze herstellt.

Das erfindungsgemäße Verfahren kann durch das folgende Formelschema (Beispiel Permethrinsäure(ester)) erläutert werden :

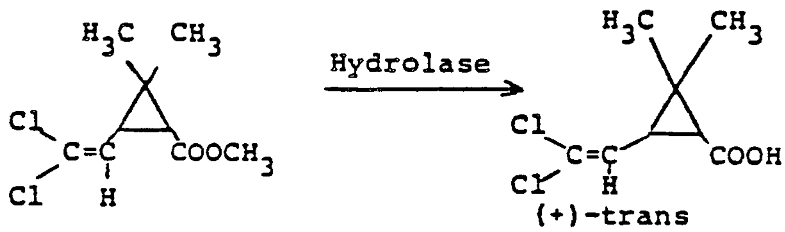


(+)-trans

(±)-trans und (±)-cis Gemische

(die nicht hydrolysierten (-)-trans und (±)-cis-Ester werden verworfen)

Weiterhin wurde gefunden, daß man die für die Herstellung der (+)-trans-Cyclopropancarbonsäuren und ihren Salzen der allgemeinen Formel (I) geeigneten Hydrolasen (Esterasen) erhält, wenn man Mikroorganismen unter den üblichen Bedingungen kultiviert, die (±)-trans-Verbindungen (gegebenenfalls in Mischung mit den (±)-cis-Verbindungen) der allgemeinen Formel II mit dem Fermentationsgut behandelt und die Stämme (oder Stammgemische) mit ausreichender Hydrolaseaktivität ausliest und die ungeeigneten Mikroorganismen verwirft.

Obwohl stereospezifische Reaktionen in der Biochemie häufig zu finden sind, war es dennoch überraschend, daß nach dem erfindungsgemäßen Verfahren die gewünschten (+)-trans-Cyclopropancarbonsäuren in glatter Weise stereoselektiv in den Isomerengemischen aus den (±)-trans-Cyclopropancarbonsäureestern der Formel II (gegebenenfalls in Mischung mit den (±)-cis-Verbindungen) gebildet und leicht von den verbleibenden Estern abgetrennt werden können.

In den allgemeinen Formeln (I) und (II) bedeutet $C_1$-$C_4$-Alkyl R geradkettiges oder verzweigtes Alkyl, wie Methyl, Ethyl, n- und i-Propyl, sowie n-, iso-, sec.- und tert.-Butyl. Vorzugsweise bedeutet $C_1$-$C_4$-Alkyl R Methyl oder Ethyl, besonders bevorzugt Methyl.

Halogen R bedeutet Fluor, Chlor, Brom und Jod, vorzugsweise Chlor und Brom, insbesondere Chlor.

Als Metallionen $R^1$ stehen vorzugsweise Alkali- oder Erdalkalionen, wie Natrium-, Kalium- oder Calciumionen.

Bevorzugt stehen in den allgemeinen Formeln (I) und (II) :

R für Chlor oder Methyl und

$R^1$ für Wasserstoff und

$R^2$ für Methyl oder Ethyl.

Von besonderem Interesse ist die erfindungsgemäße Herstellung von (+)-trans-Permethrinsäure (allgemeine Formel (I) : $R = Cl$ und $R^1 = H$) aus (±)-trans (gegebenenfalls in Mischung mit (±)-cis) Permethrinsäuremethylester (allgemeine Formel (II) : $R = Cl$ und $R^2 = CH_3$) oder von (+)-trans-Chrysanthemumsäure (allgemeine Formel (I) : $R = CH_3$ und $R^1 = H$) aus (±)-trans-(gegebenenfalls in Mischung mit (±)-cis)-Chrysanthemumsäureethylester (allgemeine Formel (II) : $R = CH_3$ und $R^2 = C_2H_5$).

In der « RS-Nomenklatur » können die sterischen Verhältnisse in den Verbindungen der allgemeinen Formeln (I) und (II), wie folgt angegeben werden :

« (±)-trans » entspricht « 1R trans » oder « 1R 3S »
« (±)-cis » entspricht dem gemisch aus « 1R 3R » und « 1S 3S »
« (±)-trans » entspricht dem Gemisch aus «1 R 3S » und « 1S 3R »
« (±)-trans und (±)-cis » Gemisch entspricht « 1RS 3RS » (Gemisch).

Bei der Auswahl geeigneter Hydrolasen (Esterase) bildender Mikroorganismen kommen praktisch alle Typen von Mikroorganismen infrage, wobei es ohne Belang ist, ob es sich dabei um prokaryotische oder eukaryotische Mikroorganismen handelt, sofern diese nur in der Lage sind, die geeigneten Hydrolasen zu erzeugen und Verbindungen der allgemeinen Formel (II) enantioselektiv zu spalten.

Bevorzugt werden Pilze der Gattungen Acremonium, Alternaria, Aspergillus, Fusarium, Mucor, Paecilomyces, Phoma, Stemphylium sowie andere Zygomycota, Ascomycota, Basidiomycota oder Deuteromycota, oder aber Bakterien der Gattungen Arthrobacter, Bacillus, Corynebacterium, Nocardia, Pseudomonas, Rhodococcus oder Streptomyces.

Ganz besonders bevorzugt werden die Stämme Paecilomyces lilacinus R 10076, Aspergillus ustus PTB 646 A und Pseudomonas testosteroni ZP 50 sowie diejenigen Varianten und Mutanten dieser Stämme, welche die für die Durchführung der vorliegenden Erfindung wesentlichen Merkmale aufweisen.

Die Stämme R 10076, PTB 646 A und ZP 50 wurden bei der Deutschen Sammlung von Mikroorganismen (DSM), Grisebachstr. 8, 3400 Göttingen, Bundesrepublik Deutschland, hinterlegt :

| Stammkurzbezeichnung | Hinterlegung : Aktenzeichen/Datum |
|---|---|
| R 10076 | DSM 2934/02.04.1984 |
| PTB 646 A | DSM 2935/02.04.1984 |
| ZP 50 | DSM 2936/02.04.1984 |

Beschreibung von Paecilomyces lilacinus R 10076

Der Stamm wurde aus einer Bodenprobe isoliert und erhielt die interne Bezeichnung R 10076.

Der Pilz bildet auf Malzagar bei 25 °C innerhalb 14 Tagen Kolonien mit einem Durchmesser von 5-7 cm ; die Kolonien sind rötlich-violett, die Unterseite ist ungefärbt. Die Konidientrager haben Phialiden und sind meist einzeln angeordnet, ihre Länge beträgt 400-600 µm.

Die Konidien sind elliptisch bis spindelförmig, glatt bis leicht rauh, 2,5-3,0 × 2,0-2,2 µm.

Der Stamm R 10076 gehört zweifelsfrei zur Art Paecilomyces lilacinus (Thom) Samson 1974.

Beschreibung von Pseudomonas testosteroni ZP 50

Stäbchenförmiges, gram-negatives Bakterium, 0,7-0,8 µm dick und 2,1-2,9 µm lang.

Beweglich mit polarer multitricher Begeißelung.

Organische Wachstumsfaktoren werden nicht benötigt.

Kohlenhydrate, wie z. B. Glucose oder Fructose werden nicht verwertet. Die optimale Wachstumstemperatur liegt bei 30 °C.

Beschreibung von Aspergillus ustus PTB 646 A

Der Pilz bildet auf Malzagar zunächst weiße bis hellgraue, später dunkelgraue bis schwarze, langsam wachsende Kolonien.

Die Konidiophoren sind dunkel, bis 400 µm larıg ; die Vesikel sind rund und ebenfalls gefärbt.

Die Konidien sind rund mit warziger Oberfläche, ihr Druchmesser beträgt ca. 4 µm.

Der Stamm PTB 646 A gehört somit zur Art Aspergillus ustus (Bain.) Thom & Church 1926.

Das erfindungsgemäße Fermentationsverfahren zur Herstellung der Hydrolase (Esterase) enthaltenden Zubereitungen kann mit Hilfe fester, halbfester oder flüssiger Nährmedien durchgeführt werden. Bevorzugt werden wässerig-flüssige Nährmedien verwendet.

Die Beimpfung der Nährmedien erfolgt nach allgemein üblichen Methoden, z. B. über Sporensuspensionen, Schrägröhrchen oder Kolbenkulturen.

Die Kultur erfolgt unter aeroben oder anaeroben Bedingungen und kann gemäß den allgemein üblichen Methoden wie unter Verwendung von Schüttelkulturen z. B. in Schüttelkolben von luftbewegten Kulturen oder von Submerskulturen durchgeführt werden. Bevorzugt erfolgt die Kultivierung im aeroben Submersverfahren in belüfteten Fermentern, z. B. in üblichen gerührten Submersfermentern. Es ist möglich, die Kultur kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen verwendet werden. Das Nährmedium muß einer oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können.

Als Kohlenstoffquellen kommen alle üblichen Kohlenstoffquellen in Frage. Beispielsweise seien Kohlenhydrate, insbesondere Polysaccharide, wie Stärke oder Dextrine, Disaccharide, wie Maltose oder Rohrzucker, Monosaccharide, wie Glucose oder Xylose, Zuckeràlkohole, wie Mannit oder Glycerin sowie natürlich vorkommende Gemische, wie Malzextrakt, Melasse oder Molkepulver genannt. Auch Kohlenwasserstoffe können dem Nährmedium zugefügt werden. Als Stickstoffquellen kommen alle üblichen organischen und anorganischen Stickstoffquellen infrage. Beispielsweise seien Eiweißstoffe, Eiweißhydrolysate, Aminosäuren, wie Glutaminsäure, Asparaginsäure, Fleischmehl, Fleischhydrolysate sowie Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt sowie Ammoniumsalze und Nitrate, z. B. $NH_4Cl$, $(NH_4)_2SO_4$, Harnstoff, $NaNO_3$ und $KNO_3$ aufgeführt. Die Mineralsalze, welche im Nährmedium enthalten sein sollten, liefern z. B. folgende Ionen :

$$Mg^{++}, Na^{+}, K^{+}, Ca^{++}, NH_4^{+}, Cl^{-}, SO_4^{--}, PO_4^{---} \text{ und } NO_3^{-}$$

sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 3 % Stickstoffquellen und vorzugsweise etwa 0,001 bis 0,5 %, insbesondere 0,003 bis 0,3 % Mineralsalze.

Über die zum Wachstum der Mikroorganismen nötigen Bestandteile der Nährlösungen können weitere Zusätze zu den Medien gemacht werden, die zu einer Erhöhung der Rate der Enzymbildung durch die Mikroorganismen oder zu einer Erhöhung der volumetrischen Gesamtausbeute an Enzym führen können. Auch können solche Substanzen zu einer Verbesserung der Gewinnung des Enzyms führen, oder dessen Freisetzung erleichtern, oder aber zur Stabilisierung der Zellen dienen, wenn ganze Zellen in freiem oder immobilisiertem Zustand für die Umsetzung eingesetzt werden.

Bei den genannten Nährmedienzusätzen kann es sich um Emulgatoren und oberflächenaktive Substanzen handeln, wie z. B. Tween 80, Brij 58, Benzylalkohol, Phenethylalkohol, Triton X 100 sowie aliphatische Alkohole verschiedener Kettenlänge und Kohlenwasserstoffe.

Der pH-Wert der wachsenden Kulturen sollte vorzugsweise zwischen etwa 5 und etwa 10, insbesondere zwischen 6,5 und 9,5 gehalten werden. Ein zu starker pH-Abfall in den sauren Bereich kann durch Zusätze einer organischen oder anorganischen Base, vorzugsweise von $CaCO_3$ vermieden werden.

Wie in der Fermentationstechnologie üblich, kann auch eine automatische pH-Regulierung durchgeführt werden, bei der sterile organische oder anorganische Säure, z. B. $H_2SO_4$, oder sterile Lauge, z. B. NaOH in Abständen in die Kulturlösung eingespritzt wird.

Es ist zweckmäßig sicherzustellen, daß die Mikroorganismen ausreichend mit Sauerstoff (bei aerobisch kultivierten Mikroorganismen) sowie den Nährstoffen in Kontakt gebracht werden. Dies kann nach den allgemein üblichen Methoden wie Schütteln und Rühren erfolgen.

Die Züchtungstemperatur kann zwischen etwa 15 und etwa 40 °C, vorzugsweise zwischen 20 und 35 °C liegen, besonders bevorzugt liegt sie bei etwa 26 °C. Die Dauer der Züchtung kann stark variiert werden, wobei z. B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 10, vorzugsweise etwa 3 bis 5 Tage nach Züchtungsbeginn erreicht. Die gewünschte Esterase-Aktivität der Fermentation kann mit Hilfe von spektrophotometrischen, flüssigchromatographischen und gaschromatographischen Methoden quantifiziert werden.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtungen können z. B. Dampf- als auch die Trockensterilisation verwendet werden, wobei die Temperaturen vorzugsweise bei 100 bis 140 °C, insbesondere bei 120 bis 130 °C liegen können.

Falls bei der Kultivierung in unerwünschter Menge Schaum entsteht, können die üblichen chemischen Schaumdämpfungsmittel, z. B. flüssige Fette und Öle, Öl-Wasser-Emulsionen, Paraffine, höhere Alkohole, wie Octadecanol, Siliconöle, Polyoxyethylen- bzw. Polyoxypropylenverbindungen (z. B. in Mengen bis etwa 1 %) zugesetzt werden. Schaum kann auch mit Hilfe der Üblichen mechanischen Vorrichtungen (welche z. B. Zentrifugalkräfte benutzen) gedämpft oder beseitigt werden.

Wie bereits oben angedeutet, können anhand eines einfachen Screening-Verfahrens die für die Herstellung der (+)-trans-Verbindungen geeigneten Mikroorganismen leicht ausgewählt werden. Hierzu werden verfügbare Mikroorganismen Stämme in üblicher Weise z. B. in einem Schüttelkolben herangezogen. Vom Fermentationsgut werden die Zellen abgetrennt. Ein kleiner Teil (z. B. 1 ml) der Fermentationsbrühe wird mit Substrat (z. B. 1 ml einer Emulsion von 2,23 mg (±)-trans-Permethrinsäuremethylester in 1 ml 0,1 M Phosphatpuffer) versetzt und bei pH 7 ca. 15 Stunden bei etwa 30 °C geschüttelt. Durch Zugabe von Säure (z. B. 0,1 ml 5 N HCl) wird die Reaktion beendet, die wässrige Reaktionsmischung mit Essigsäureethylester extrahiert und der Gehalt an gewünschtem Produkt durch Hochdruckflüssigkeitschromatographie (HPLC) bestimmt. Auf diese Weise kann das Vorliegen von extrazellulärer Hydrolase festgestellt werden. Zur Bestimmung einer zellgebundenen Hydrolase wird entsprechend eine wässrige Aufschwemmung der abgetrennten und gewaschenen Zellen (z. B. Myzel) mit dem Substrat versetzt. Mit Hilfe dieser einfachen Methode können geeignete Mikroorganismen ausgesucht werden, Optimierungen im Hinblick auf das eingesetzte Substrat vorgenommen werden und auch bestimmt werden ob die Hydrolase extrazellulär und/oder zellgebunden vorliegt.

Das Verfahren der Aufarbeitung des Fermentationsgutes und der Hydrolyse der (+)-trans (gegebenenfalls in Mischung mit (±)-cis) Verbindungen der allgemeinen Formel (II) mit nachfolgender Abtrennung, wird zweckmäßigerweise wie folgt, durchgeführt, wobei sich unterschiedliche Verfahrensmaßnahmen ergeben, wenn die Hydrolase (Esterase) zellfrei oder zellgebunden oder sowohl zellfrei als auch zellgebunden vorliegt.

Zur Gewinnung der Zubereitung einer extrazellulären Hydrolase wird nach Beendigung der Fermentation die Fermentationsbrühe nach den üblichen Methoden (z. B. durch Zentrifugation) von den Zellen abgetrennt. Aus der erhaltenen zellfreien Fermentationsbrühe wird das Enzym (neben anderen Eiweißbestandteilen) ebenfalls nach üblichen Methoden (z. B. Zugabe von Alkoholen, wie Methanol oder Ethanol oder von anorganischen Salzen, wie Ammoniumsulfat) ausgefällt. Der Niederschlag wird (z. B. durch Zentrifugation) abgetrennt und in einer Pufferlösung (z. B. Phosphatpuffer) aufgelöst. Diese Lösung kann direkt für die Durchführung des Hydrolyseschrittes verwendet werden. Im Falle, daß das Enzym zellgebunden vorliegt, können die abgetrennten Zellen in einer Pufferlösung aufgeschwemmt und direkt für die Hydrolyse eingesetzt werden. Normalerweise ist es nicht erforderlich eine weitere Reinigung dieser Enzymzubereitungen vorzunehmen. Eine weitere Aufarbeitung der Enzymzubereitungen kann jedoch nach den allgemein üblichen Methoden leicht erfolgen (z. B. Umfällungsmethoden, chromatographische Methoden, Freisetzung von zellgebundenem Enzym durch Zellzertrümmerung u. s. w.) und ist besonders dann wünschenswert, wenn das Enzym nach den üblichen Methoden in modifizierter Form, z. B. an feste anorganische oder organische Träger (z. B. Zeolithe, Polysaccharide, Polyamide, Polystyrolharze, Polyacrylharze u.s.w.) gebunden oder mikroverkapselt eingesetzt werden soll (immobilisiertes Enzym).

Die Umsetzung der (±)-trans Verbindungen (gegebenenfalls in Mischung mit den (±)-cis Isomeren) der allgemeinen Formel (II) (im folgenden kurz « Cyclopropancarbonsäureester » genannt) in die (+)-trans Verbindungen der allgemeinen Formel (I) (im folgenden kurz « Cyclopropancarbonsäuren » genannt) mit Hilfe der obigen Enzymzubereitungen wird zweckmäßigerweise, wie folgt, vorgenommen :

In dem Fall, in dem das Enzym extrazellulär, also in wässriger Lösung vorliegt, wird eine Emulsion oder eine Suspension der Cyclopropancarbonsäureester in einer wässrigen Puffer-Lösung mit der Enzymzubereitung versetzt und die Mischung gerührt. Die Hydrolysereaktion wird durch hochdruckflüssigkeitschromatographische oder gaschromatographische Untersuchungen von Proben verfolgt, welche von Zeit zu Zeit entnommen werden. Der pH-Bereich kann je nach Substrat und Enzym über weite Bereiche schwanken. Man arbeitet vorzugsweise zwischen pH 5 und pH 10, besonders bevorzugt zwischen pH 6 und 9 und ganz besonders bevorzugt zwischen pH 6,5 und 8,8. Die pH-Werte können durch die Verwendung geeigneter Puffersysteme (z. B. Phosphatpuffer oder Tris-Puffer) leicht eingestellt, bzw. eingehalten werden. Die Reaktionstemperaturen liegen zwischen 15 und 80 °C, vorzugsweise zwischen 20 und 40 °C und besonders bevorzugt zwischen 25 und 35 °C.

Zur Beendigung der Reaktion wird durch Säurezugabe (z. B. $H_2SO_4$ oder von HCl) der pH-Wert stark sauer (z. B. pH 1 bis 2, vorzugsweise 1,2 bis 1,8) eingestellt. Das Reaktionsgemisch wird anschließend mit organischen Lösungsmitteln, welche mit Wasser schwer mischbar sind, wie gegebenenfalls halogenierten aliphatischen oder aromatischen Kohlenwasserstoffen, z. B. Methylenchlorid, Estern, z. B. Essigsäureethylester oder Ethern, z. B. Diethylether oder deren Gemische, extrahiert. Die organische Phase ( im folgenden auch als « organische Phase I » bezeichnet) wird für die weitere Aufarbeitung abgetrennt und die wässrige Phase verworfen.

Für den Fall, in dem die Hydrolase zellgebunden vorliegt, wird entsprechend vorgegangen. Hier wird abweichend von der obigen Verfahrensweise lediglich anstelle der wässrigen Lösung der Hydrolase eine Suspension der Zellen (oder des Mycels) in Wasser eingesetzt, das Reaktionsgemisch wird bis zum Abbruch der Reaktion geschüttelt und die Zellen werden anschließend abgetrennt (z. B. durch Zentrifugation). Aus dem wässrigen Reaktionsgemisch wird die « organische Phase I », wie oben beschrieben, erhalten.

Zur weiteren Aufarbeitung wird die « organische Phase I » mit einer wässrigen Alkalilösung (z. B. von Natrium- oder Kaliumhydroxid, Natrium- oder Kaliumhydrogencarbonat oder Natrium- oder Kaliumcarbonat) versetzt, welche einen pH-Wert zwischen 8 und 10 aufweist. Das Gemisch wird zur Extraktion geschüttelt, die wässrige Phase abgetrennt und die verbleibende organische Phase (gegebenenfalls nach Wiederholung der Extraktion) verworfen. Anschliessend wird die erhaltene wäßrige Lösung angesäuert (z. B. mit einer 5 N HCl auf pH 1 bis 3) und mit einem Mit Wasser schwer mischbaren organischen Lösungsmittel versetzt, wobei die gleichen Lösungsmittel herangezogen werden können, welche oben bei der Herstellung der « organischen Phase I » angeführt werden. Die Mischung wird zur Extraktion geschüttelt und die organische Phase zur weiteren Aufarbeitung abgetrennt (organische Phase II) und die wässrige Phase verworfen. Durch Messung des Drehwertes kann die optische Reinheit der erhaltenen Cyclopropancarbonsäuren bestimmt werden, welche in üblicher Weise, durch Abdestillieren des Lösungsmittels isoliert werden können. Gewünschtenfalls können durch die Umsetzung mit Basen (z. B. Alkali- oder Erdalkalihydroxiden oder Carbonaten) die Cyclopropancarbonsäuresalze erhalten werden.

Das erfindungsgemäße Verfahren kann in entsprechender Weise nach den üblichen Methoden auch mit Hilfe der modifizierten (z. B. immobilisierten) Enzyme durchgeführt werden.

Biochemische Verfahrensweisen und Methoden, welche im Rahmen der erfindungsgemäßen Verfahrens angewendet werden können, werden in Bryan Williams and Keith Wilson, Principles and Techniques of Practical Biochemistry, Edward Arnold (Publishers) Ltd., London, 1975 und entsprechende deutsche Ausgabe Praktische Biochemie Georg Thieme Verlag, Stuttgart, 1978 beschrieben, wo sich auch viele Hinweise auf weitere Literatur findet.

Benutzte Materialien und Warenzeichen :

Tween 80 ist ein Polyoxyethylen-sorbitan.
Tween ist ein Warenzeichen der Firma ICI America Inc., Atlas Chemicals Division, USA.
Brij 58 ist ein Polyoxyethylen-monocetylether.
Brij ist ein Warenzeichen der Firma ICI America Inc., Atlas Chemicals Division, USA.
Triton X-100 ist ein p-t-Octylphenyl-polyethylenglykolether.
Triton ist ein Warenzeichen der Firma Rohm & Haas, USA.
Baylith T 144 ist ein Zeolith.
Baylith ist ein Warenzeichen der Bayer AG, Leverkusen, Bundesrepublik Deutschland.
Bacto Pepton ist ein Eiweißhydrolysat der Difco Laboratories, Detroit, USA.

Das erfindungsgemäße Verfahren soll durch die folgenden Beispiele erläutert werden (wo nichts anderes angegeben ist, entsprechen alle %-Angaben Gewichtsprozenten) :

### Beispiel 1
### (Screening-Methode)

Die Fermentation der zu prüfenden Mikroorganismen wird in 1 Liter Kolben in üblicher Weise durchgeführt. Nach dem Absetzen der Zellen wird 1 ml der Fermentationsbrühe mit 1 ml einer Emulsion von 2,23 mg (±)-trans-Permethrinsäuremethylester (Verbindung der Formel (II) mit R = Cl und $R^2 = CH_3$) in 0,1 molarem Phosphatpuffer versetzt und das Gemisch (pH 7) für 12 Stunden bei 30 °C geschüttelt. Anschließend wird mit 0,1 ml 5 N HCl versetzt und mit 2 ml Essigsäureethylester extrahiert. Die organische

Phase wird HPLC-analytisch (reversed phase chromatography) an einer RP 10-Säule (der Fa. Merck, Darmstadt, Bundesrepublik Deutschland) mit UV-Detektor untersucht und somit festgestellt, ob und inwieweit der jeweilige Stamm Hydrolasen erzeugt, welche das Ausgangsprodukt in die (+)-trans Permethrinsäure (Verbindung der Formel (I) mit R = Cl und $R^1$ = H) überführen. In entsprechender Weise wird zum Auffinden von zellgebundenem Hydrolasen statt der Kulturbrühe 1 ml einer wässrigen Zellsuspension eingesetzt.

Beispiel 2
(Hydrolase aus Paecilomyces lilacinus R 10076)

In einem 1 000 ml Erlenmeyerkolben werden 100 ml eines Kulturmediums, das 3 % Sojamehl, 2 % Glucose und 0,5 % $K_2HPO_4$ enthält, durch Erhitzen auf die Siedetemperatur sterilisiert.

Nach der Sterilisation wird der pH-Wert des Mediums mit steriler 1 M Salzsäure und steriler 1 M Natronlauge auf pH 7,0 eingestellt.

Die Beimpfung des Mediums erfolgt mit Hilfe einer Impföse von einer Schrägröhrchen-Kultur des Stammes Paecilomyces lilacinus R 10076 aus.

Die Kultivierung dauert 120 Stunden bis zum Erreichen einer hohen enzymatischen Aktivität und wird bei 26 °C auf einer Schüttelmaschine durchgeführt. Das Mycel wird aus der Kulturbrühe abzentrifugiert; diese wird mit 70 ml (±)-trans-Permethrinsäuremethylester-Emulsion versetzt (2.23 mg/ml (±)-trans-Permethrinsäuremethylester in 0.1 M Phosphatpuffer, pH 8.0). Die Reaktionsmischung wird bei 30 °C unter Schütteln 20 Stunden inkubiert. Danach säuert man durch Zugabe von 5 N HCl auf pH 1.5 an, und extrahiert mit 100 ml $CH_2Cl_2$ (organische Phase I). Anschließend schüttelt man die « organische Phase I » mit 100 ml 0.1 N $Na_2CO_3$. Die wässrige $Na_2CO_3$-Phase wird dann mit 5 N HCl auf pH 1,5 gestellt, und mit 100 ml $CH_2Cl_2$ extrahiert (« organische Phase II »).

Die « organische Phase II » wird mit $Na_2SO_4$ getrocknet; ihr Gehalt an (+)-trans-Permethrinsäure flüssigchromatographisch bestimmt und der Drehwert gemessen. Gehalt an (+)-trans-Permethrinsäure: 31.2 mg ; $\alpha_D^{20}$ = + 0.048 ; Optische Reinheit 70 %.

Beispiel 3
(Hydrolase aus Aspergillus ustus PTB 646 A)

In einem 1 000 ml Erlenmeyerkolben werden 100 ml eines Kulturmediums, das 2 % Hefeextrakt, 2 % Glycerin und 0,25 % $CaCO_3$ enthält, durch Erhitzen zum Sieden sterilisiert.

Nach der Sterilisation wird der pH-Wert des Mediums mit steriler 1 M Salzsäure und steriler 1 M Natronlauge auf pH 7.0 eingestellt.

Die Beimpfung des Mediums erfolgt mit Hilfe einer Impföse von einer Schrägröhrchen-Kultur des Stamms Aspergillus ustus PTB 646 A aus.

Die Kultivierung dauert 72 Stunden bis zum Erreichen einer hohen enzymatischen Aktivität und wird bei 26 °C auf einer Schüttelmaschine durchgeführt. Das Enzym befindet sich dann zu 70 % innerhalb der Zellen.

Die Zellen werden abzentrifugiert (10 g feuchte Masse), und mit 70 ml Puffer (0.1 Mol/1 Tris, pH 8.2) und 200 g Glasperlen (Korngröße 0.075-0.150 mm) vermischt.

Der Aufschluß der Zellen wird unter Wasserkühlung in einer « Vibrogen-Zellmühle Vi 4 » (Fa. Bühler, Tübingen) durchgeführt (15 min.), danach die Suspension klar zentrifugiert. 10.0 ml des Überstands werden mit 10 ml einer Suspension von (±)-trans-Permethrinsäuremethylester (10 mMol/l) in Tris-Puffer (0.1 Mol/l, ph 8.2) versetzt und bei 30 °C inkubiert. Die Reaktion wird nach 16 h durch Zugabe von 2 ml 12 N $H_2SO_4$ gestoppt, und mit 1 Vol. Teil Essigsäureethylester extrahiert.

Die organische Phase wird mittels HPLC analysiert; man findet 13.77 μ Mol trans-Permethrinsäure (13.8 % Umsatz). Durch fraktionierte Extraktion wird die Säure isoliert. Hierzu wird die Essigsäureethylester-Phase mit 0,1 m $Na_2CO_3$ extrahiert. Die abgetrennte organische Phase wird verworfen. Die wässrige Phase wird mit verdünnter $H_2SO_4$ auf pH 2 angesäuert und anschließend mit Essigsäureethylester extrahiert. Die Essigsäureethylesterphase wird abgetrennt und das Lösungsmittel abdestilliert. Die gewünschte Säure bleibt als Rückstand zurück. Sie weist einen Drehwert von $[\alpha]_D$ = + 0.140 (c = 0.46, EtOH) auf. Die optische Reinheit beträgt 82 %.

Beispiel 4
(Hydrolase aus Pseudomonas testosteroni ZP 50)

In einem 1 000 ml Erlenmeyerkolben werden 100 ml eines Kulturmediums, das 3 % Bacto Pepton, 1,5 % Hefeextrakt und 0,5 % $K_2HPO_4$ enthält, durch Erhitzen zum Sieden sterilisiert.

Die Beimpfung des sterilisierten Mediums erfolgt mit Hilfe einer Impföse von einer Schrägröhrchenkultur des Stammes Pseudomonas testosteroni ZP 50.

Die Kultivierung dauert 36 Stunden bis zum Erreichen einer hohen enzymatischen Aktivität und wird bei 26 °C auf einer Schüttelmaschine durchgeführt. Das Enzym befindet sich überwiegend im Innern der Zellen.

Die Zellen werden abzentrifugiert (10 g Feuchtgewicht) und in 70 ml 0,1 M Phosphat-Puffer, pH 8,5, suspendiert und mit 200 g Glasperlen (0,075-0,150 mm Durchmesser) vermischt. Durch Schütteln in einer Zellmühle (15 min) (Typ « Vibrogen », Fa. Bachofen) werden die Zellen zerstört und die Zelltrümmer abzentrifugiert.

Der Überstand wird auf trans-Permethrinsäuremethylester-Esterase-Aktivität geprüft. Dazu wird eine Suspension von (±)-trans-Permethrinsäuremethylester (10 mMol/l) in Phosphat-Puffer (0,1 Mol/l, pH 8,5) hergestellt und 1,0 ml des Zellrohsaftes mit 1,0 ml der Estersuspension gemischt. Bei 35 °C wird 1 h inkubiert, dann stoppt man ab mit 100 µl 12 N $H_2SO_4$ und extrahiert mit 2,0 ml Essigsäureethylester. Die organische Phase wird mittels HPLC auf den Gehalt an (±)-trans-Permethrinsäuremethylester bzw. Permethrinsäure analysiert.

Resultat : 1 ml des Zellrohsaftes hydrolysiert 0,01 µ Mol (±)-trans-Permethrinsäuremethylester in der Minute.

Der Gehalt an (+)-Enantiomeren in der Säure wird gas-chromatographisch bestimmt. Er beträgt 99 % (+)-Entiomer.

Beispiel 5

(Immobilisierung der Esterase)

10 g Zellen von Pseudomonas testosteroni ZP 50 werden in 70 ml Phosphat-Puffer (0,1 Mol/l, pH 8,5) suspendiert und mit 200 g Glasperlen in einer Zellmühle (Typ « Vibrogen ») aufgeschlossen. Die Mischung wird zentrifugiert, und der Überstand mit Ammoniumsulfat (bis 40 % der Sättigungskonzentration) versetzt. Der entstandene Niederschlag wird verworfen. Der resultierende Überstand wird mit weiteren Ammoniumsulfat versetzt, bis 60 % der Sättigungskonzentration.

Der entstandene Niederschlag, der das Enzym enthält, wird abzentrifugiert und in 10 ml Phosphat-Puffer (0,1 Mol/l, pH 7,0) gelöst. Über Nacht wird gegen 10 l Phosphat-Puffer (0,1 Mol/l, pH 7,0) dialysiert.

10,0 g Zeolith Typ « Baylith T 144 » werden nach einer Standardmethode silanisiert (H. H. Weetall : « Covalent Coupling Methods for Inorganic Support Materials » in : S. P. Colowick, N. O. Kaplan (eds.) : Methods in Enzymology, Vol. XLIV, S. 140). Hierzu wird der Zeolith mit 40 ml 3-Aminopropyltriethoxysilan (10 %ige Lösung in Wasser) (Firma Ega-Chemie, Bundesrepublik Deutschland) versetzt, der pH auf 3,5 eingestellt mittels 6 N HCl und die Mischung im Schüttelwasserbad bei 75 °C 20 min inkubiert. Danach wird abgesaugt, mit Wasser gewaschen und 5 h bei 115 °C getrocknet.

Der Zeolith wird jetzt mit 250 ml Glutardialdehyd versetzt (2,5 %ige Lösung in Phosphat-Puffer, 0,05 Mol/l, pH 7,0) und 90 min geschüttelt. Danach wird abfiltriert, mit Wasser gewaschen, und mit der dialysierten Enzymlösung versetzt (10 ml). Es wird 4 h geschüttelt, abgesaugt und mit Phosphat-Puffer (0,1 Mol/l, pH 7,0) gewaschen.

Das immobilisierte und das freie Enzym werden getestet, indem einerseits 1,0 g enzymbeladener Zeolith mit 1,0 ml Phosphat-Puffer (s. o.) und andererseits 1,0 ml der dialysierten Enzymlösung mit 1,0 ml (±)-trans-Permethrinsäuremethylester-Suspension vermischt werden und bei 35 °C 1 h inkubiert werden. Die Teste werden mit 12 N $H_2SO_4$ gestoppt, mit 2,0 ml Essigsäureethylester extrahiert und mittels HPLC analysiert.

Ergebnis :

| | |
|---|---|
| Aktivität des freien Enzyms | 0,11 µMol/(min × ml) |
| Aktivität des immobilisierten Enzyms | 0,012 µMol/(min × g) |
| Ausbeute an aktivem Enzyme 10,9 % | |

Beispiel 6

(Hydrolyse von (+)-Chrysanthemumsäure-Ethylester mit Pseudomonas testosteroni ZP 50)

1,5 g Zellen (Feuchtgewicht) von Pseudomonas testosteroni ZP 50 werden in 3.0 ml Phosphatpuffer (0.1 Mol/l, pH 8.5) suspendiert. 6.0 mg (±)-cis, trans-Chrysanthemumsäure-Ethylester ($R = CH_3$ und $R^2 = C_2H_5$) werden in 3.0 ml Phosphat-Puffer (0.1 Mol/l, pH 8.5) unter Zusatz von Brij 58 (80 mg/l) emulgiert. Die Lösungen werden gemischt und 16 h bei 30 °C unter Schütteln inkubiert.

Danach werden 300 µl 12 N Schwefelsäure zugesetzt, und weiter 6.0 ml Ethylacetat zur Extraktion.

Die Ethylacetat-Phase wird abgetrennt und hochdruckflüssigchromatografisch analysiert. Man findet 0.16 mg/ml (±)-Chrysanthemumsäure ($R = CH_3$ und $R^1 = H$). Weiterhin wird die Ethylacetat-Lösung mit 1 Vol. Natriumcarbonat-Lösung (0.1 Mol/l) extrahiert. Nach Phasentrennung wird die wäßrige Phase auf pH 1.5 gestellt, und mit 1 Vol. Chloroform extrahiert. Die Phasen werden getrennt, die organische Phase auf 1.5 ml eingestellt, und die optische Drehung an Polarimeter gemessen. $\alpha = + 0.07$ ($c = 0.06$).

**Patentansprüche**

1. Verfahren zur Herstellung von (+)-trans-Cyclopropancarbonsäuren oder ihrer Salze der allgemeinen Formel (I)

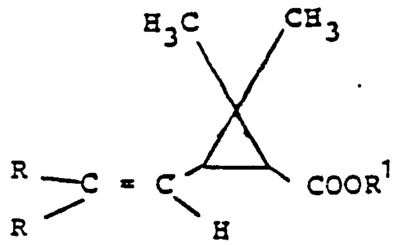

(I)

in welcher

R für Halogen oder $C_1$-$C_4$-Alkyl steht und

$R^1$ für Wasserstoff oder das Äquivalent eines Metallions steht,

und wobei in Formel (I) die sterischen Verhältnisse nicht wiedergegeben werden, dadurch gekennzeichnet, daß man die (±)-trans-Cyclopropancarbonsäureester der allgemeinen Formel (II), gegebenenfalls in Mischung mit den entsprechenden (±)-cis-Cyclopropancarbonsäureestern der allgemeinen Formel (II)

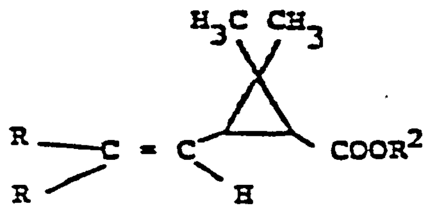

(II)

in welcher

R die bei der allgemeinen Formel (I) angegebene Bedeutung hat und

$R^2$ für Methyl oder Ethyl steht, wobei in Formel (II), die sterischen Verhältnisse nicht wiedergegeben werden,

mit Hydrolasen mikrobieller Herkunft, welche extrazellulär und/oder zellulär, gegebenenfalls immobilisiert, vorliegen, behandelt und die entstandenen (+)-trans-Cyclopropancarbonsäuren der allgemeinen Formel (I) nach den üblichen Methoden isoliert und gegebenenfalls reinigt und gegebenenfalls die Salze herstellt.

2. Verfahren gemäß Anspruch 1, dadurch gekennzeichnet, daß in der allgemeinen Formel (I)

R für Chlor oder Methyl steht und

$R^1$ für Wasserstoff oder das Äquivalent eines Metallions steht.

3. Verfahren gemäß Anspruch 1, dadurch gekennzeichnet, daß als Verbindung der allgemeinen Formel (II), (±)-trans (gegebenenfalls in Mischung mit (±)-cis) permethrinsäuremethylester eingesetzt wird und als Verbindung der allgemeinen Formel (I) (+)-trans-Permethrinsäure erhalten wird.

4. Verfahren gemäß Anspruch 1, dadurch gekennzeichnet, daß als Verbindung der allgemeinen Formel (II), (±)-trans (gegebenenfalls in Mischung mit (±)-cis) Chrysanthemumsäureethylester eingesetzt wird und als Verbindung der allgemeinen Formel (I) (+)-trans-Chrysanthemumsäure erhalten wird.

5. Verfahren gemäß den Ansprüchen 1 bis 4, dadurch gekennzeichnet, daß die Hydrolysereaktion bei pH-Werten zwischen 6 und 9 durchgeführt wird.

6. Verfahren gemäß den Ansprüchen 1 bis 5, dadurch gekennzeichnet, daß die Hydrolysereaktion bei Temperaturen zwischen 25 und 80 °C durchgeführt wird.

7. Verfahren gemäß den Ansprüchen 1 bis 6, dadurch gekennzeichnet, daß Hydrolasen verwendet werden, welche durch die Mikroorganismenstämme R 10076 (DSM 2934), PTB 646 A (DSM 2935) und ZP 50 (DSM 2936) oder durch ihre Mutanten und Varianten erzeugt werden.

8. Verfahren gemäß den Ansprüchen 1 bis 7, dadurch gekennzeichnet, daß die Hydrolasen in immobilisierter Form vorliegen.

9. Mikroorganismenstämme R 10076 (DSM 2934) und PTB 646 A (DSM 2935) sowie ihre Mutanten und Varianten, die Hydrolasen für das Verfahren gemäß Anspruch 1 herstellen können.

10. Verwendung von Hydrolasen mikrobieller Herkunft zur Herstellung von (+)-trans-Cyclopropancarbonsäuren der allgemeinen Formel I gemäß Anspruch 1, aus (±)-trans-Cyclopropancarbonsäureestern der allgemeinen Formel II gemäß Anspruch 1, welche gegebenenfalls auch (±)-cis-Cyclopropancarbonsäureester enthalten.

## Claims

1. Process for the preparation of (+)-trans-cyclopropanecarboxylic acids or their salts of the general formula (I)

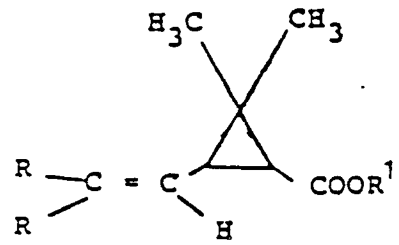

(I)

in which

R represents halogen or $C_1$-$C_4$-alkyl, and

$R^1$ represents hydrogen or the equivalent of a metal ion,

and where the steric relationships are not represented in formula (I), characterised in that the (±)-trans-cyclopropanecarboxylic esters of the general formula (II), where appropriate mixed with the corresponding (±)-cis-cyclopropanecarboxylic esters of the general formula (II)

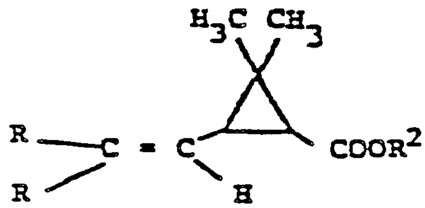

(II)

in which

R has the meaning indicated for the general formula (I) and

$R^2$ represents methyl or ethyl,

the steric relationships not being represented in formula (II), are treated with hydrolases which are of microbial origin and which are extracellular and/or cellular, where appropriate immobilised, and the resulting (+)-trans-cyclopropanecarboxylic acids of the general formula (I) are isolated and, where appropriate, purified by customary methods and, where appropriate, the salts are prepared.

2. Process according to Claim 1, characterised in that in the general formula (I)

R represents chlorine or methyl, and

$R^1$ represents hydrogen or the equivalent of a metal ion.

3. Process according to Claim 1, characterised in that methyl (±)-trans (where appropriate mixed with (±)-cis) permethrate is used as the compound of the general formula (II), and (+)-trans-permethric acid is obtained as the compound of the general formula (I).

4. Process according to Claim 1, characterised in that ethyl (±)-trans (where appropriate mixed with (±)-cis) chrysanthemate is used as the compound of the general formula (II), and (+)-trans-chrysanthemic acid is obtained as the compound of the general formula (I).

5. Process according to Claims 1 to 4, characterised in that the hydrolysis reaction is carried out at pH values between 6 and 9.

6. Process according to Claims 1 to 5, characterised in that the hydrolysis reaction is carried out at temperatures between 25 and 80 °C.

7. Process according to Claims 1 to 6, characterised in that the hydrolases used are produced by the microorganism strains R 10076 (DSM 2934), PTB 646 A (DSM 2935) and ZP 50 (DSM 2936) or by their mutants and variants.

8. Process according to Claims 1 to 7, characterised in that the hydrolases are in an immobilised form.

9. Microorganism strains R 10076 (DSM 2934) and PTB 646 A (DSM 2935) and their mutants and variants which are capable of producing hydrolases for the process according to Claim 1.

10. Use of hydrolases of microbial origin for the preparation of (+)-trans-cyclopropanecarboxylic acids of the general formula I according to Claim 1 from (±)-trans-cyclopropanecarboxylic esters of the general formula II according to Claim I, which, where appropriate, also contain (±)-cis-cyclopropanecarboxylic esters.

**Revendications**

1. Procédé de production d'acides (+)-trans-cyclopropanecarboxyliques ou de leurs sels de formule générale (I)

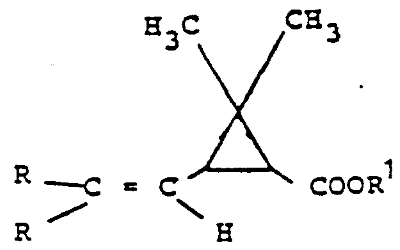

(I)

dans laquelle

R est un halogène ou un groupe alkyle en $C_1$ à $C_4$ et

$R^1$ est l'hydrogène ou l'équivalent d'un ion métallique,

et dans la formule (I), les conditions stériques ne sont pas reproduites, caractérisé en ce qu'on traite les esters d'acides (±)-trans-cyclopropanecarboxyliques de formule générale (II), éventuellement en mélange avec les esters correspondants d'acides (±)-cis-cyclopropanecarboxyliques de formule générale (II)

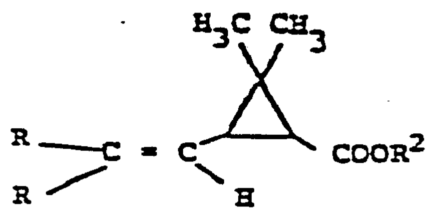

(II)

dans laquelle

R a la définition indiquée à propos de la formule générale (I) et

$R^2$ représente un groupe méthyle ou éthyle, les conditions stériques n'étant pas reproduites pour la formule (II),

avec des hydrolases d'origine microbienne qui sont extracellulaires et/ou cellulaires, éventuellement immobilisées, et on isole les acides (+)-trans-cyclopropanecarboxyliques produits de formule générale (I) selon des procédés connus et, le cas échéant, on les purifie et on prépare éventuellement les sels.

2. Procédé suivant la revendication 1, caractérisé en ce que dans la formule générale (I),

R représente le chlore ou le groupe méthyle et

$R^1$ représente l'hydrogène ou l'équivalent d'un ion métallique.

3. Procédé suivant la revendication 1, caractérisé en ce qu'on utilise, comme composé de formule générale (II), l'ester méthylique de l'acide de (±)-trans-perméthrine (le cas échéant en mélange avec la formule (±-cis) et on obtient comme composé de formule générale (I) l'acide de (±)-trans-perméthrine.

4. Procédé suivant la revendication 1, caractérisé en ce qu'on utilise, comme composé de formule générale (II), l'ester éthylique de l'acide (±)-trans-chrysanthémique (éventuellement en mélange avec la forme (±)-cis) et on obtient comme composé de formule générale (I) l'acide (+)-trans-chrysanthémique.

5. Procédé suivant les revendications 1 à 4, caractérisé en ce que la réaction d'hydrolyse est conduite à des valeurs de pH comprises entre 6 et 9.

6. Procédé suivant les revendications 1 à 5, caractérisé en ce que la réaction d'hydrolyse est conduite à des températures comprises entre 25 et 80 °C.

7. Procédé suivant les revendications 1 à 6, caractérisé en ce qu'on utilise des hydrolases qui sont produites par les souches de micro-organismes R 10076 (DSM 2934), PTB 646 A (DSM 2935) et ZP 50 (DSM 2936) ou par leurs mutants et variants.

8. Procédé suivant les revendications 1 à 7, caractérisé en ce que les hydrolases se présentent sous la forme immobilisée.

9. Les souches de micro-organismes R 10076 (DSM 2934) et PTB 646 A (DSM 2935) ainsi que leurs mutants et variants, qui peuvent produire des hydrolases pour le procédé suivant la revendication 1.

10. Utilisation d'hydrolases d'origine microbienne pour la préparation d'acides (+)-trans-cyclopropanecarboxyliqués de formule générale I suivant la revendication 1, à partir d'esters d'acides (±)-trans-cyclopropanecarboxyliques de formule générale (II) suivant la revendication 1, qui contiennent aussi le cas échéant des esters d'acides (±)-cis-cyclopropanecarboxyliques.